(19) **Europäisches Patentamt**

**European Patent Office**

**Office européen des brevets**

(11) **EP 1 141 370 B1**

(12) **EUROPÄISCHE PATENTSCHRIFT**

(45) Veröffentlichungstag und Bekanntmachung des Hinweises auf die Patenterteilung:
**25.06.2003 Patentblatt 2003/26**

(51) Int Cl.[7]: **C12P 19/18**, C08B 37/00, A61K 9/20
// C12P19:18, C12R1:36

(21) Anmeldenummer: **99962188.1**

(22) Anmeldetag: **30.11.1999**

(86) Internationale Anmeldenummer:
**PCT/EP99/09299**

(87) Internationale Veröffentlichungsnummer:
**WO 00/039321 (06.07.2000 Gazette 2000/27)**

(54) **ALPHA-1,4-GLUCANKETTEN ENTHALTENDE POLYSACCHARIDE SOWIE VERFAHREN ZU IHRER HERSTELLUNG**

POLYSACCHARIDES CONTAINING ALPHA-1,4-GLUCAN CHAINS AND METHOD FOR PRODUCING SAME

POLYSACCHARIDES CONTENANT DES CHAINES DE ALPHA-1,4-GLUCANE ET PROCEDE PERMETTANT DE LES PREPARER

(84) Benannte Vertragsstaaten:
**AT BE CH CY DE DK ES FI FR GB GR IE IT LI LU MC NL PT SE**

(30) Priorität: **28.12.1998 DE 19860376**

(43) Veröffentlichungstag der Anmeldung:
**10.10.2001 Patentblatt 2001/41**

(73) Patentinhaber: **Celanese Ventures GmbH 65926 Frankfurt am Main (DE)**

(72) Erfinder:
- **WEISSMÜLLER, Max**
  **D-65830 Kriftel (DE)**
- **QUANZ, Martin**
  **D-10557 Berlin (DE)**
- **PROVART, Nicholas**
  **D-14163 Berlin (DE)**

(74) Vertreter: **Luderschmidt, Schüler & Partner Patentanwälte Industriepark Höchst 65926 Frankfurt (DE)**

(56) Entgegenhaltungen:
WO-A-95/31553          US-A- 5 229 277

- OKADA G ET AL: "NEW STUDIES ON AMYLOSUCRASE, A BACTERIAL ALPHA-D-GLUCOSYLASE THAT DIRECTLY CONVERTS SUCROSE TO A GLUCOGEN-LIKE ALPHA-GLUCAN" JOURNAL OF BIOLOGICAL CHEMISTRY,US,THE AMERICAN SOCIETY OF BIOLOGICAL CHEMISTS, INC.,, Bd. 249, Nr. 1, 10. Januar 1974 (1974-01-10), Seiten 126-135, XP000867741 ISSN: 0021-9258 in der Anmeldung erwähnt
- DATABASE BIOLOGICAL ABSTRACTS [Online] Abstract PREV199800075745, Dezember 1997 (1997-12) XP002134915 & LEE C Y ET AL: "Production of glucooligosaccharides by an acceptor reaction using two types of glucansucrase from Streptococcus sobrinus" BIOTECHNOLOGY LETTERS, Bd. 19, Nr. 12, Dezember 1997 (1997-12), Seiten 1227-1230, KEW, SURREY, GB ISSN: 0141-5492

**Beschreibung**

Die vorliegende Erfindung betrifft $\alpha$-1,4-Glucanketten enthaltende Polysaccharide sowie ein Verfahren zu ihrer Herstellung.

Polysaccharide sind Polymere, die sich aus zahlreichen glykosidisch verbundenen Monosacchariden zusammensetzen. Polysaccharide kommen sowohl in höheren Lebewesen als auch in Mikroorganismen wie Bakterien vor und erfüllen dort beispielsweise die Funktion von Speicher- und Gerüstsubstanzen. Kommerzielle Verwendung finden die Polysaccharide u.a. als Hilfs- und Zusatzstoffe in der Lebensmittelindustrie, in der Leichtindustrie, im Gesundheitswesen und in der Analytik.

Glucane sind Polysaccharide, die ausschließlich aus Glucosemonomeren bestehen. In den $\alpha$-1,4-Glucanen sind diese Glucosereste durch $\alpha$-1,4-glykosidische Bindungen miteinander verknüpft. $\alpha$-1,4-Glucane können aufgrund ihrer physikalisch-chemischen Eigenschaften zur Herstellung von Folien verwendet werden, die farb-, geruch- und geschmacklos, nichttoxisch und biologisch abbaubar sind. Schon heute gibt es für solche Folien zahlreiche Anwendungsmöglichkeiten z.B. in der Lebensmittelindustrie, der Textilindustrie und der Glasfaserindustrie.

Das am häufigsten vorkommende natürliche $\alpha$-1,4-Glucan ist Amylose, ein Bestandteil der Stärke. Amylose wird bereits zur Herstellung von Fasern verwendet, deren Eigenschaften denen natürlicher Zellulosefasern ähneln und deren teilweisen oder vollständigen Ersatz bei der Papierherstellung ermöglichen. In der Pharmazie wird Amylose als Füllmittel für Tabletten, für Pasten und als Zusatz zu Hautschutzstoffen verwendet. In der Nahrungsmittelindustrie dient sie als Dickungs-und Bindemittel für Puddings, Suppen, Saucen, Mayonnaisen, Cremefüllungen und als Gelatineersatz. Amylose wird ferner als Bindemittel bei der Herstellung von schallisolierenden Wandvertäfelungen verwendet.

Amylopektin, der Hauptbestandteil der Stärke, und Glykogen sind weitere Polysaccharide, deren Hauptketten aus Glucoseresten mit $\alpha$-1,4-glykosidischer Verknüpfung bestehen. Diese Polysaccharide tragen Seitenketten, die mit der Hauptkette über $\alpha$-1,6-glykosidische Bindungen verknüpft sind. Auch diese Polysaccharide finden in der Industrie ebenfalls in großem Umfang Verwendung.

Die Isolierung von $\alpha$-1,4-Glucanen wie Stärke und Glykogen aus pflanzlichen und tierischen Lebewesen ist aufwendig und kostspielig und führt nicht immer zu Produkten mit reproduzierbaren Eigenschaften. Aus diesem Grunde haben Bakterien, die solche Glucane produzieren können, zunehmend Aufmerksamkeit gefunden.

In den meisten Bakterien erfolgt die Synthese von Polysacchariden in ähnlicher Weise wie in höheren Lebewesen über Nukleotid-aktivierte Zucker. So sind beispielsweise an der Biosynthese von Glykogen in den meisten Bakterien drei Enzyme beteiligt, nämlich ADP-Glucose-Phosphorylase, die die Bildung von ADP-Glucose aus Glucose-1-phoshat und ATP katalysiert, Glykogen-Synthase, die die Glucose von ADP-Glucose auf die wachsende Glucankette überträgt, und ein Verzweigungsenzym, das $\alpha$-1,6-Verknüpfungen in die lineare $\alpha$-1,4-Glucankette einführt. In einigen Bakterien kann die Polysaccharidsynthese jedoch auch ohne die Beteiligung aktivierter Zucker erfolgen.

Eines der bakteriellen Systeme, das Polysaccharide ohne Beteiligung von Nukleotidzuckern zu synthetisieren vermag, wurde in Bakterien der Gattung *Neisseria* gefunden. In diesen Bakterien werden Polysaccharide mit einer ähnlichen Struktur wie Glykogen durch das Enzym Amylosaccharase direkt aus Saccharose, dem natürlichen Substrat des Enzyms, synthetisiert [Okada, G., und E.J. Hehre, J. Biol. Chem. 249:126-135 (1974); MacKenzie, C.R. et al, Can. J. Microbiol. 23:1303-1307 (1977); MacKenzie, C.R. et al, Can. J. Microbiol. 24:357-362 (1978)]. Amylosaccharase (Saccharose: 1,4-$\alpha$-Glucan 4-$\alpha$-Glucosyltransferase, E.C. 2.4.1.4.) katalysiert die Bildung von $\alpha$-1,4-glykosidisch verknüpften Glucanen, indem es unter Freisetzung von D-Fructose den Glucosylrest des Saccharosemoleküls gemäß dem folgenden Reaktionsschema

$$\text{Saccharose} + (\alpha\text{-1,4-D-Glucosyl})_n \longrightarrow \text{D-Fructose} + (\alpha\text{-1,4-D-Glucosyl})_{n+1}$$

auf die wachsende Polymerkette überträgt. Nukleotid-aktivierte Zucker oder Cofaktoren werden bei dieser Reaktion nicht benötigt. Das Enzym wird jedoch durch die Anwesenheit von Glucosylgruppenakzeptoren (oder Primern), auf die der Glucosylrest der Saccharose gemäß dem obigen Reaktionsschema unter $\alpha$-1,4-Glucan-Kettenverlängerung übertragen wird, beispielsweise von Oligo- und Polysacchariden wie Amylose- oder Glykogen, stimuliert [Okada, G., und E.J. Hehre, J. Biol. Chem. 249:126-135 (1974); Remaud-Simeon, M. et al., *In* S.B. Petersen, B. Svenson und S. Pedersen (Hrsg.), Carbohydrate bioengineering, S. 313-320 (1995);. Elsevier Science B.V., Amsterdam, Niederlande].

Amylosaccharasen wurden bislang lediglich in Bakterien der Gattung *Neisseria* gefunden. Das Enzym, das in den Bakterien konstitutiv exprimiert wird, ist äußerst stabil und bindet sehr fest an sein Polymerisationsprodukt. In den meisten untersuchten Spezies ist das Enzym intrazellulär lokalisiert, in *Neisseria polysaccharea* wird die Amylosucrase jedoch sekretiert. Das Gen für Amylosaccharase aus *Neisseria polysaccharea* konnte inzwischen isoliert und mit gentechnischen Methoden exprimiert werden. Es wurde gefunden, daß das Enzym mit hoher Wahrscheinlichkeit ausschließlich die Bildung von linearen $\alpha$-1,4-Glucanketten katalysiert (WO 95/31553).

Die Verwendung von Amylosaccharase aus *N. polysaccharea* zur Herstellung von linearen $\alpha$-1,4-Glucanen

wurde bereits in der WO 95/31553 vorgeschlagen. Ein Problem bei der Verwendung von Amylosaccharasen zur Herstellung von Polysacchariden besteht jedoch darin, daß die unter der Einwirkung von Amylosaccharase üblicherweise gebildeten Polysaccharide sehr unterschiedliche Molekulargewichte, also eine hohe Polydispersität oder breite Molekulargewichtsverteilung, aufweisen. Wegen ihrer homogeneren physikalisch-chemischen Eigenschaften sind für eine industrielle Anwendung jedoch Polysaccharidpräparationen mit einem möglichst einheitlichen Molekulargewicht, also niedriger Polydispersität, erwünscht.

Aufgabe der vorliegenden Erfindung war es daher, $\alpha$-1,4-Glucanketten enthaltende Polysaccharide mit niedriger Polydispersität bereitzustellen.

Diese Aufgabe wurde mit den in den Ansprüchen wiedergegebenen Verfahren und Polysacchariden gelöst.

Gegenstand der vorliegenden Erfindung ist demnach ein Verfahren, worin ein Glucosylgruppenakzeptor durch Umsetzung mit Saccharose in Gegenwart einer Amylosaccharase einer Kettenverlängerungsreaktion unterworfen wird, wobei die Menge an Glucosylgruppenakzeptor im Reaktionsgemisch so gewählt ist, daß das Molverhältnis von zur Kettenverlängerung zur Verfügung stehenden Enden des Glucosylgruppenakzeptors zu Saccharose wenigstens 1 :1000 und/oder das Gewichtsverhältnis von Glucosylgruppenakzeptor zu Saccharose wenigstens 1 : 50 beträgt.

Ein weiterer Gegenstand der vorliegenden Erfindung ist ein Verfahren, worin ein Glucosylgruppenakzeptor durch Umsetzung mit Saccharose in Gegenwart einer Amylosaccharase und unter Zusatz von Fructose einer Kettenverlängerungsreaktion unterworfen wird.

$\alpha$-1,4-Glucanketten enthaltende Polysaccharide, die nach diesen Verfahren erhältlich sind, sind ebenfalls Gegenstand dieser Erfindung.

Bei den erfindungsgemäß verwendeten Glucosylgruppenakzeptoren handelt es sich um Verbindungen, an denen unter Amylosaccharase-katalysiertem Transfer von aus Saccharose stammenden $\alpha$-D-Glucosylresten eine Synthese von $\alpha$-1,4-Glucanketten, also eine a-1,4-Glucankettenverlängerung erfolgen kann. Geeignete Glucosylgruppenakzeptoren sind insbesondere kurz- und längerkettige Oligo-und Polysaccharide mit endständigen, über $\alpha$-1,4-glycosidische Bindungen verknüpften Glucoseresten. Bevorzugt ist der erfindungsgemäß verwendete Glucosylgruppenakzeptor ein lineares, besonders bevorzugt ein verzweigtes Oligo- oder Polysaccharid. Beispiele für erfindungsgemäße Glucosylgruppenakzeptoren sind Maltooligosaccharide wie Maltopentaose, Maltohexaose oder Maltohepatose.

Bevorzugte Glucosylgruppenakzeptoren sind Dextrine, Amylopektine, Amylosen und Amylose-ähnliche Polysaccharide, beispielsweise aus Mais und Kartoffeln, sowie Glykogene und Glykogen-ähnliche Polysaccharide, beispielsweise aus Muskelgewebe, Muscheln oder Bakterien.

Besonders bevorzugte Glucosylgruppenakzeptoren sind verzweigte Polysaccharide wie Glycogen. Solche verzweigten Glucosylgruppenakzeptoren besitzen mehr als ein Ende, an dem eine Kettenverlängerung stattfinden kann. So trägt die Glycogenkette ungefähr 7-12% Verzweigungen, auf die Glucosylreste übertragen werden können.

Überraschend wurde nun gefunden, daß sich bei der Amylosaccharase-katalysierten Synthese von $\alpha$-1,4-Glucanen Polysaccharide mit niedriger Polydispersität erhalten lassen, wenn das Molverhältnis von zur Kettenverlängerung zur Verfügung stehenden Enden des Glucosylgruppenakzeptors zu Saccharose und/oder das Gewichtsverhältnis von Glucosylgruppenakzeptor zu Saccharose im Reaktionsgemisch einen bestimmten Mindestwert annimmt. Vermutlich ist die Kettenverlängerungsreaktion bei diesem Mindestwert gegenüber Nebenreaktionen, die Polysaccharidpräparationen hoher Polydispersität zur Folge haben, bevorzugt. Dabei nimmt die Polydispersität bei gleichbleibender Saccharosekonzentration mit steigender Konzentration des Akzeptors tendenziell ab.

Zweckmäßig beträgt das Molverhältnis von zur Kettenverlängerung zur Verfügung stehenden Enden des Glucosylgruppenakzeptors zu Saccharose im Reaktionsansatz wenigstens 1 : 1000. Bevorzugt beträgt das Molverhältnis wenigstens 5 : 1000 und besonders bevorzugt wenigstens 1 : 100. Die obere Grenze für das Molverhältnis von zur Kettenverlängerung zur Verfügung stehenden Enden des Glucosylgruppenakzeptors zu Saccharose ist nicht sehr kritisch und beträgt zweckmäßig ungefähr 1 : 50 bis 1 : 25.

Das Gewichtsverhältnis von Glucosylgruppenakzeptor zu Saccharose beträgt zweckmäßig wenigstens 1 : 50, beispielsweise wenigstens 2 : 50 oder wenigstens 5 : 50. Das optimale Gewichtsverhältnis hängt von der Art des Akzeptors ab. Bei verzweigten Polysaccharidakzeptoren ist die in der Reaktionsmischung benötigte Menge an Akzeptor bei einer gegebenen Saccharosekonzentration in der Regel niedriger als bei unverzweigten oder lediglich geringverzwigten Polysaccharidakzeptoren. So hat sich bei der Verwendung von Glykogen mit einem gewichtsmittleren Molekulargewicht $M_w$ von ca. 160.000 g/mol ein Verhältnis von Akzeptor zu Saccharose von wenigstens 2,5 : 50 als vorteilhaft erwiesen, während bei Dextrinen mit einem $M_w$ von ungefähr 5000 bis 6000 g/mol ein Gewichtsverhältnis von Akzeptor zu Saccharose von wenigstens 5 : 50 bis 10 : 50 bevorzugt wird.

Bei einer gegebenen Saccharosekonzentration und einem gegebenen Glucosylgruppenakzeptor ist das Molekulargewicht des nach dem erfindungsgemäßen Verfahren erhaltenen Polysaccharids umso niedriger, je höher die Konzentration an Glucosylgruppenakzeptor gewählt wird. Auf diese Weise läßt sich durch geeignete Wahl des Gewichtsverhältnisses von Glucosylgruppenakzeptor zu Saccharose auch das Molekulargewicht des Endprodukts steuern.

Die absolute Konzentration der als Substrat der Amylosaccharasen im Reaktionsansatz eingesetzten Saccharose ist nicht kritisch. Die eingesetzte Menge überschreitet zweckmäßig aber nicht 50% (w/v), da oberhalb dieser Konzentration die Viskosität der Lösung zu hoch ist und die Reaktionsgeschwindigkeit stark abnimmt. Vorzugsweise liegt die Saccharosekonzentration im Reaktionsansatz zwischen 1 und 30% (w/v).

Die für die Kettenverlängerungsreaktion optimalen Bedingungen wie z.B. Molverhältnis von zur Kettenverlängerung zur Verfügung stehenden Enden des Glucosylgruppenakzeptors zu Saccharose, Gewichtsverhältnis von Glucosylgruppenakzeptor zu Saccharose und Saccharosekonzentration im Reaktionsgemisch lassen sich durch einfache Versuche ohne weiteres ermitteln.

Es wurde ferner gefunden, daß sich bei der Amylosaccharase-katalysierten Synthese von $\alpha$-1,4-Glucanen Polysaccharide mit niedriger Polydispersität erhalten lassen, wenn dem Reaktionsansatz Fructose zugesetzt wird. Vermutlich inhibiert ein Fructosezusatz störende Nebenreaktionen, die Polysaccharidpräparationen hoher Polydispersität zur Folge haben. Der durch die Anwesenheit von Fructose hervorgebrachte Effekt wird unabhängig davon beobachtet, ob die Mol- und Gewichtsverhältnisse von Glucosylgruppenakzeptor zu Saccharose die oben angegebenen Mindestwerte aufweisen oder nicht. Die Zugabe von Fructose führt zu einer noch engeren Molekulargewichtsverteilung, also noch niedrigeren Polydispersität, des erhaltenen Endprodukts, dafür ist die Ausbeute jedoch etwas geringer.

Zweckmäßig wird Fructose dem Reaktiongemisch in einer Konzentration von wenigstens 10mM zugesetzt. Bevorzugt wird die Fructose in einer Konzentration von wenigstens 50mM, vorzugsweise von 100 bis 800mM, zugesetzt

Mit dem erfindungsgemäßen Verfahren läßt sich ohne weiteres eine Zunahme des Molekulargewichts des eingesetzten Glucosylgruppenakzeptors auf das Zwei- bis Dreifache erreichen. Das Molekulargewicht des in dem erfindungsgemäßen Verfahren eingesetzten Akzeptors hängt daher auch vom gewünschten Molekulargewicht des Endprodukts ab. Da die Reaktionsgeschwindigkeit der Kettenverlängerungsreaktion mit steigendem Polymerisationsgrad des Akzeptors zunimmt, werden jedoch zweckmäßig Akzeptoren mit einem gewichtsmittleren Molekulargewicht $M_w$ von wenigstens $0,5 \times 10^3$ g/mol, vorzugsweise von wenigstens $4 \times 10^3$ g/mol und besonders bevorzugt von wenigstens $1 \times 10^5$ bis $1 \times 10^6$ g/mol eingesetzt. Da die Polydispersität der erhaltenen Reaktionsprodukte auch durch die Einheitlichkeit des eingesetzten Akzeptormaterials beeinträchtigt wird, empfiehlt es sich außerdem, Akzeptormoleküle mit möglichst niedriger Polydispersität einzusetzen.

Als Amylosaccharasen können alle Enzyme eingesetzt werden, die gemäß dem Reaktionsschema

$$\text{Saccharose} + (\alpha\text{-1,4-D-Glucosyl})_n ---> \text{D-Fructose} + (\alpha\text{-1,4-D-Glucosyl})_{n+1}$$

in der Lage sind, den Glucosylrest eines Saccharosemoleküls unter Freisetzung von D-Fructose und Bildung einer $\alpha$-1,4-Glucankette auf das Akzeptormolekül zu übertragen. Bevorzugt werden Amylosaccharasen aus Prokaryonten, insbesondere aus Bakterien der Gattung *Neisseria*, eingesetzt. Geeignet sind beispielsweise die in den Bakterienspezies *N. sicca*, *N. canis*, *N. cinerea, N. perflava*, *N. subflava*, *N. denitrificans* und *N. polysaccharea* vorkommenden Amylosaccharasen. Bevorzugt wird Amylosaccharase aus *N. polysaccharea,* beispielsweise aus *N. polysaccharea* ATCC 43768, eingesetzt.

Die verwendeten Amylosaccharasen können entweder unmittelbar aus den Organismen isoliert werden, in denen sie natürlicherweise synthetisiert werden (MacKenzie, C.R. et al., Can. J. Microbiol., 24: 357-362; 1978), oder sie können, wie in der WO 95/31553 beschrieben, mit gentechnischen Methoden (rekombinante Amylosaccharasen) hergestellt werden. Die Enzyme können auch zellfrei unter Verwendung von in vitro-Transkriptions- und Translationssystemen hergestellt werden.

Die Amylosaccharasen können sowohl als Rohenzyme oder in partiell aufgereinigter Form als auch in hoch gereinigter Form eingesetzt werden. Bevorzugt werden hoch gereinigte Amylosaccharasen eingesetzt, wobei unter dem Begriff "hoch gereinigte Amylosaccharase" insbesondere eine Amylosaccharase mit einem Reinheitsgrad von wenigstens 80%, bevorzugt von wenigstens 90% und besonders bevorzugt von wenigstens 95% verstanden wird.

Der Einsatz hoch gereinigter Amylosaccharasen in dem erfindungsgemäßen Verfahren hat den Vorteil, daß die Enzyme keine Reste des Stammes, beispielsweise des Mikroorganismus, enthalten, aus denen sie isoliert wurden. Beispielsweise enthalten hoch gereinigte Präparate keine anderen unerwünschten Enzyme, beispielsweise Polysaccharide abbauende Enzymen wie Amylasen. Der Einsatz hoch gereinigter Amylosaccharasen ist auch für die Anwendung in der Lebensmittel- und Pharmaindustrie vorteilhaft, da ein definiertes und von unnötigen Bestandteilen befreites Reaktionsmedium auch ein genauer definiertes Produkt liefert. Dies führt zu weniger aufwendigen Zulassungsverfahren für diese biotechnologisch erzeugten Produkte in der Lebensmittel- und Pharmaindustrie, insbesondere wenn diese Produkte keine Spuren transgener Mikroorganismen aufweisen sollen.

Bevorzugt werden rekombinante Amylosaccharasen eingesetzt, wie sie beispielsweise in der WO 95/31553 beschrieben werden. Solche rekombinanten Amylosaccharasen können gegenüber den natürlich vorkommenden Amylosaccharasen gegebenenfalls auch durch Mutationen, beispielsweise Insertionen, Deletionen und Substitutionen, genetisch modifiziert sein, um bestimmte Eigenschaften des exprimierten Proteins zu verändern. So kann die Amylosaccharase beispielsweise als Fusionsprotein zusammen mit einer Polypeptidsequenz exprimiert werden, deren spezifische Bindungseigenschaften eine leichtere Isolierung des Fusionsproteins, beispielsweise über Affinitätschromatographie, ermöglichen (s. z.B. Hopp et al., Bio/Technology 6 (1988), 1204-1210; Sassenfeld, Trends Biotechnol. 8 (1980), 88-93). Besonders bevorzugt werden Amylosaccharasen eingesetzt, die von den Wirtszellen in das Nährmedium sekretiert werden, so daß kein Aufschluß von Zellen und keine weitere Aufreinigung des Enzyms erforderlich ist, weil das sekretierte Enzym aus dem Überstand gewonnen werden kann. Die Sekretion der Amylosaccharase kann wie bei *N. polysaccharea* natürlich erfolgen oder die Sekretion kann dadurch erreicht werden, daß das Enzym zusammen mit einem Signalpeptid exprimiert wird, mit dessen Hilfe das Enzym die Zellmembran des Wirtsorganismus durchdringen kann.

Die Amylosaccharase kann in freier Form oder immobilisiert an einem Trägermaterial eingesetzt werden. Eine Immobilisierung der Amylosaccharase bietet den Vorteil, daß das Enzym auf einfache Weise aus dem Reaktionsgemisch wiedergewonnen und mehrfach verwendet werden kann. Da die Aufreinigung von Enzymen in der Regel kostenund zeitaufwendig ist, erlaubt eine Immobilisierung und Wiederverwertung des Enzyms eine erhebliche Kosteneinsparung. Ein weiterer Vorteil ist der Reinheitsgrad der Reaktionsprodukte, die keine Reste an Protein enthalten. Als Trägermaterialien eignen sich beispielsweise Agarose, Algenat, Cellulose, Polyacrylamid, Silica oder Nylon, wobei die Kopplung an das Trägermaterial über kovalente oder nicht-kovalente Bindung erfolgen kann.

Die Menge an eingesetzter Amylosaccharase liegt üblicherweise zwischen 0,1 und 100 U/ml, vorzugsweise zwischen 1 und 50 U/ml und besonders bevorzugt zwischen 2 und 25U/ml.

Die Herstellung der erfindungsgemäßen Polysaccharide erfolgt zweckmäßig in vitro in pufferfreien oder gepufferten wässrigen Systemen mit einem pH zwischen 4 und 9, vorzugsweise zwischen 5,5 und 7,5. Geeignete Puffersysteme sind beispielsweise Citrat-, Maleat- und Acetatpuffer.

Die Reaktionstemperatur liegt zweckmäßig zwischen 10 und 60°C, vorzugsweise zwischen 25 und 45°C.

Die Reaktion wird zweckmäßig bis zum vollständigen Umsatz der Saccharose durchgeführt. Üblicherweise liegt die Reaktionsdauer hierfür zwischen 1 und 150 Stunden, beispielsweise zwischen 10 und 100 Stunden.

Die erfindungsgemäß gebildeten Polysaccharide sind häufig in Wasser schwer löslich und lassen sich daher ohne Schwierigkeiten, beispielsweise durch Zentrifugation, aus dem Reaktionsgemisch abtrennen. In Wasser lösliche oder teilweise lösliche Polysaccharide können beispielsweise durch Fällung mit Ethanol oder durch Ausfrieren gewonnen werden.

Die erfindungsgemäßen Verfahren erlauben eine einfache und kostengünstige Herstellung von $\alpha$-1,4-Glucanketten enthaltenden Polysacchariden niedriger Polydispersität. Die Verfahren zeichen sich durch eine gute Steuerbarkeit des Molekulargewichts der Endprodukte und durch eine hervorragende Reproduzierbarkeit aus. Dies ermöglicht die Herstellung von Produkten gleichbleibender Einheitlichkeit und Reinheit und damit von hoher Qualität, die für eine weiter industrielle Nutzung von großer Bedeutung ist. Die erhaltenen Produkte lassen sich kostengünstig aufarbeiten, da die Verfahrensparameter, die für die Aufarbeitung erforderlich sind, nicht für jeden Aufarbeitungsansatz neu optimiert werden müssen.

Beschreibung der Zeichnungen:

Fig. 1 zeigt die Molmassenverteilung von $\alpha$-1,4-Glucanketten enthaltenden Polysacchariden bei der Umsetzung von Glykogen als Glucosylgruppenakzeptor mit Saccharose in Gegenwart von Amylosaccharase in Abhängigkeit von der Glykogenund der Saccharosekonzentration

Fig. 2a zeigt die Molmassenverteilung von $\alpha$-1,4-Glucanketten enthaltenden Polysacchariden bei der Umsetzung von Glykogen mit Saccharose in Gegenwart von Amylosaccharase in Abhängigkeit von der Glykogen- und der Saccharosekonzentration in An- und Abwesenheit von Fruktose.

Fig. 2b zeigt die Molmassenverteilung von $\alpha$-1,4-Glucanketten enthaltenden Polysacchariden bei der Umsetzung von Dextrin mit Saccharose in Gegenwart von Amylosaccharase in Abhängigkeit von Dextrin- und Saccharosekonzentration in Anund Abwesenheit von Fruktose.

Die vorliegende Erfindung wird durch die nachfolgenden Beispiele näher erläutert.

**Beispiel 1:**

Reinigung von Amylosaccharase

Zur Herstellung von Amylosaccharase wurden *E.coli*-Zellen verwendet, die mit dem Vektor pNB2 enthaltend eine Amylosaccharase aus *Neisseria polysaccharea* transformiert waren (WO 95/31553).

Eine Über-Nacht-Kultur dieser *E.coli*-Zellen, die die Amylosaccharase aus *Neisseria polysaccharea* sekretieren, wurde abzentrifugiert und in ca. 1/20 Volumen 50 mM Natriumcitratpuffer (pH 6,5), 10 mM DTT (Dithiothreitol), 1 mM PMSF (Phenylmethylsulfonylfluorid) resuspendiert. Anschließend wurden die Zellen mit einer French-Press bei 1,103 x 10$^8$ Pa (16.000 p.s.i.) zweimal aufgeschlossen. Danach wurden dem

Zellextrakt 1 mM MgCl$_2$ sowie Benzonase (Merck; 100.000 Units; 250 Units $\mu$l$^{-1}$) in einer Endkonzentration von 12,5 Units ml$^{-1}$ zugegeben. Anschließend wurde der Ansatz bei 37°C unter leichtem Rühren mindestens 30 min inkubiert. Der Extrakt wurde mindestens 1,5 Stunden auf Eis stehen gelassen. Anschließend wurde 30 min bei ca. 40.000 g zentrifugiert, bis der Überstand relativ klar war. Es wurde eine Vorfiltration mit einer PVDF Membrane (Millipore "Durapore", o.ä.) durchgeführt, die einen Porendurchmesser von 0,45 $\mu$m besaß. Der Extrakt wurde über Nacht bei 4°C stehen gelassen. Zur Durchführung der HI-(hydrophobic interaction)-Chromatographie wurde der Extrakt mit festem NaCl versetzt, und auf eine Konzentration von 2 M NaCl eingestellt. Anschließend wurde wiederum für 30 min bei 4°C und ca. 40 000 g zentrifugiert. Danach wurde der Extrakt von letzten Resten an E.coli befreit, indem er mit einer PVDF Membrane (Millipore "Durapore" o.ä.) filtriert wurde, die einen Porendurchmesser von 0,22 $\mu$m aufwies. Der filtrierte Extrakt wurde über eine Butylsepharose-4B-Säule (Pharmacia) aufgetrennt (Volumen der Säule: 93 ml, Länge: 17,5 cm). Ca. 50 ml Extrakt mit einer Amylosaccharase-Aktivität von 1 bis 5 Units$^{-1}$ wurden auf die Säule gegeben. Anschließend wurden mit 150 ml Puffer B nicht-bindende Proteine von der Säule (Puffer B; 50 mM Natriumcitrat pH 6,5, 2M NaCl) gewaschen. Die Amylosaccharase wurde schließlich mit Hilfe eines fallenden, linearen NaCl-Gradienten eluiert (von 2 M bis zu 0 M NaCl in 50 mM Natriumcitrat in einem Volumen von 433 ml bei einer Zuflußrate von 1,5 ml min$^{-1}$), welcher mit Hilfe eines automatischen Pumpsystems (FPLC, Pharmacia) generiert wurde. Die Elution der Amylosaccharase erfolgt zwischen 0,7 M und 0,1 M NaCl. Die Fraktionen wurden gesammelt, über eine PD10-Sephadex-Säule (Pharmacia) entsalzen, mit 8,7 % Glycerol stabilisiert, auf Amylosaccharase-Aktivität überprüft und schließlich in Lagerpuffer (8,7 % Glycerol, 50 mM Citrat) eingefroren.

**Beispiel 2:**

Bestimmung der Amylosaccharase-Aktivität

Aufgereinigtes Protein oder Proteinrohextrakt wurde in unterschiedlichen Verdünnungen in 1 ml Ansätzen, enthaltend 5 % Saccharose, 0,1 % Glycogen und 100 mM Citrat, pH 6,5, gegeben und bei 37 °C inkubiert. Nach 5 min, 10 min, 15 min, 20 min, 25 min und 30 min wurden diesem Ansatz je 100 $\mu$l entnommen und durch sofortiges Erhitzen für 10 min auf 95 °C wurde die enzymatische Aktivität der Amylosaccharase gestoppt. Mit Hilfe eines gekoppelten Enzymtest wurde die durch die Amylosaccharase aus Saccharose freigesetzte Menge Fructose photometrischen bestimmt (M. Stitt et al., Methods in Enzymology 174:518-552; 1989). Dazu werden 1 $\mu$l bis 10 $\mu$l der inaktivierten Probe in 1 ml 50 mM Imidazolpuffer pH 6,9, 2mM MgCl$_2$, 1 mM ATP, 0,4 mM NAD und 0,5 U/ml Hexokinase gegeben. Nach sequentieller Zugabe von Glucose-6-phosphat Dehydrogenase (aus Leuconostoc mesenteroides) und Phosphoglucose-Isomerase wird die Absorptionsänderung bei 340 nm gemessen. Anschließend wird mit Hilfe des Lambert-Beerschen-Gesetzes die Menge an freigesetzter Fructose berechnet. Setzt man den erhaltenen Wert mit dem Zeitpunkt der Probennahme in Beziehung, so läßt sich die Zahl der Enzymeinheiten U bestimmen.

1 U wurde als die Menge Amylosaccharase definiert, die unter den obengenannten Bedingungen 1 $\mu$mol Fructose/min freisetzt.

**Beispiel 3:**

Herstellung von Polysacchariden

3.1 Zur Herstellung von Polysaccharidpräparationen wurde Amylosaccharase in 10 ml 0,1 M Natriumacetat-Puffer, pH 6,5, 0,02% Natriumazid bei 37°C mit verschiedenen Konzentrationen an Glycogen (Merck; M$_W$ 160.000, Polydispersität ca. 1,4) als Glucosylgruppenakzeptor und Saccharose als Substrat bis zum vollständigen Umsatz der Saccharose, d.h. mindestens 48 hr, inkubiert. Die Amylosaccharase wurde in einer Konzentrationen von 5 bis 20 U/ml eingesetzt. Eine parallele Kontrollprobe ohne Glykogen wurde unter ansonsten identischen Bedingungen behandelt.

Das ausgefallene Polysaccharid wurde abzentrifugiert (15 min, 1200g) und zweimal durch Resuspension in Wasser und erneutes Zentrifugieren gewaschen. Die Pellets wurden bei -20°C eingefroren und bei 0,34 mbar und einer Umgebungstemperatur von 25°C gefriergetrocknet (Alpha 1.4 Gefriertrockner, Christ). Die Probentemperatur während des Trocknens betrug -25°C.

Die erhaltenen Produkte wurden mittels Gelpermeationchromatographie (GPC) analysiert.

Die Arbeiten wurden nach DIN 55672-1 durchgeführt. Sämtliche Messungen wurden in Dimethylsulfoxid (DMSO) mit 0,09M NaNO$_3$ als Eluent durchgeführt. Zur GPC wurde eine Säulenkombination aus PS-Gel-Säulen (10$^3$, 10$^5$

und $10^6$ Å; Fa. PSS, Mainz, Typ "SDV 10µ"). Zur Detektion der Massenanteile wurde ein Differentialrefraktometer der Fa. Shodex, Typ "RI 71" verwendet. Es wurde eine Pumpe der Fa. Bischoff, Typ "HPLC Compact Pump" verwendet. Die Flußgeschwindigkeit betrug 1ml/min. Es wurden linear Pullulane der Fa PSS, Mainz, zur Kalibrierung verwendet. (Da die Proben verzweigte Strukturen aufweisen, stellen die gemessenen Proben somit keine absoluten sondern relative Größen dar, die aber innerhalb eines konstanten Verzweigungsgrades der Proben vergleichbar sind.) Die verwendete GPC-Software der Fa PSS "Win-GPC scientific 4.02 war mit DIN 55672-1 konform und voll validiert. Die Richtigkeit der Datenverarbeitungsschritte konnte also systemunabhäng nachvollzogen werden. Mollmassen mit Werten unter 1000g/mol wurden bei der Auswertung nicht berücksichtigt.

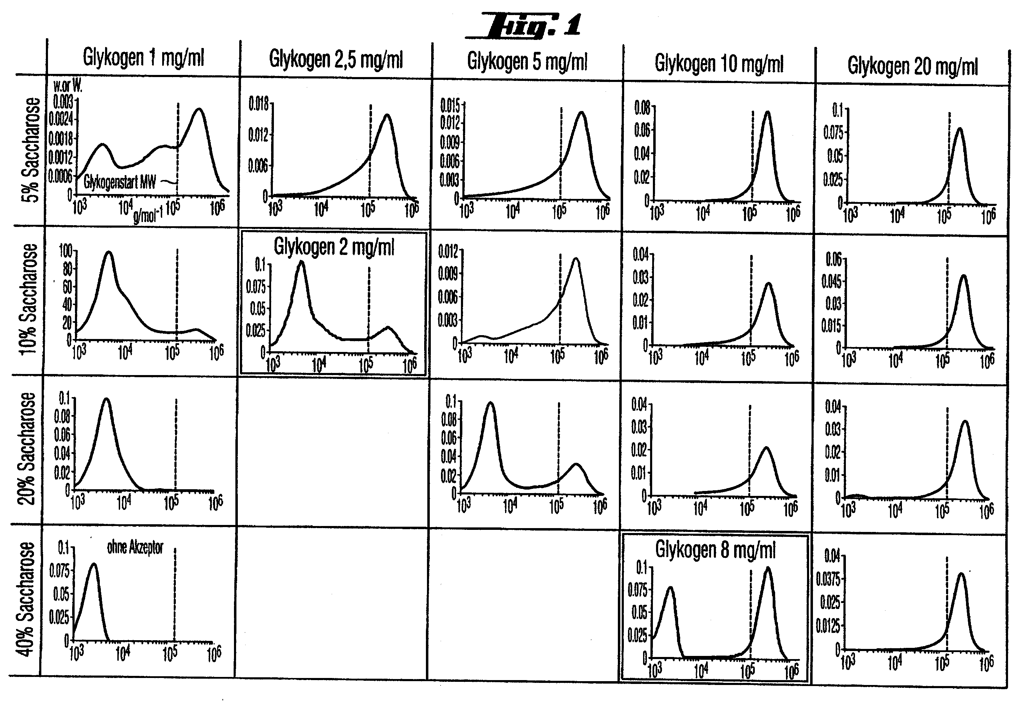

Die Ergebnisse sind in der nachfolgenden Tabelle aufgeführt und in Fig. 1 graphisch dargestellt ($w_i$ und $W_i$ bezeichnen die normierten bzw. relativen Massenanteile der i-ten Polymerfraktion). Die senkrechte gepunktete Linie zeigt das Anfangsmolekuargewicht des als Glucosylgruppenakzeptor eingesetzten Glykogens.

Die Ergebnisse zeigen, daß die Polydispersität der erhaltenen Polysaccharide bei gleichbleibender Saccharosekonzentration und steigenden Glykogenkonzentrationen drastisch sinkt.

Tabelle

| Glykog. | Saccharose (%) 5 | | | 10 | | | 20 | | | 40 | | |
|---|---|---|---|---|---|---|---|---|---|---|---|---|
| mg/ml | $M_w$ | $M_n$ | $I_n$ | $M_w$ | $M_n$ | $I_n$ | $M_w$ | $M_n$ | $I_n$ | $M_w$ | $M_n$ | $I_n$ |
| 0 | 67170 | 2129 | 32 | 7248 | 1953 | 3,7 | 4210 | 1215 | 3,5 | 2082 | 924 | 2,3 |
| 1 | 224400 | 17460 | 13 | 101700 | 7894 | 13 | 11250 | 2811 | 4 | | | |
| 2,5 | 251400 | 28080 | 9 | 95670 | 4701 | 20 | | | | | | |
| 5 | 315300 | 71650 | 4,4 | 248500 | 24785 | 10,7 | 113300 | 5580 | 21,5 | | | |
| 10 | 303500 | 235600 | 1,3 | 316900 | 76380 | 4,1 | 247900 | 12380 | 20 | 200700 | 5069 | 40 |
| 20 | 244100 | 195600 | 1,3 | 317000 | 243600 | 1,3 | 319400 | 40560 | 7,9 | 277800 | 20090 | 14 |

$M_w$ Gewichtsmittel des Molekulargewichts

$M_n$ Zahlenmittel des Molekulargewichts

$I_n$ Polydispersität ($M_w/M_n$)

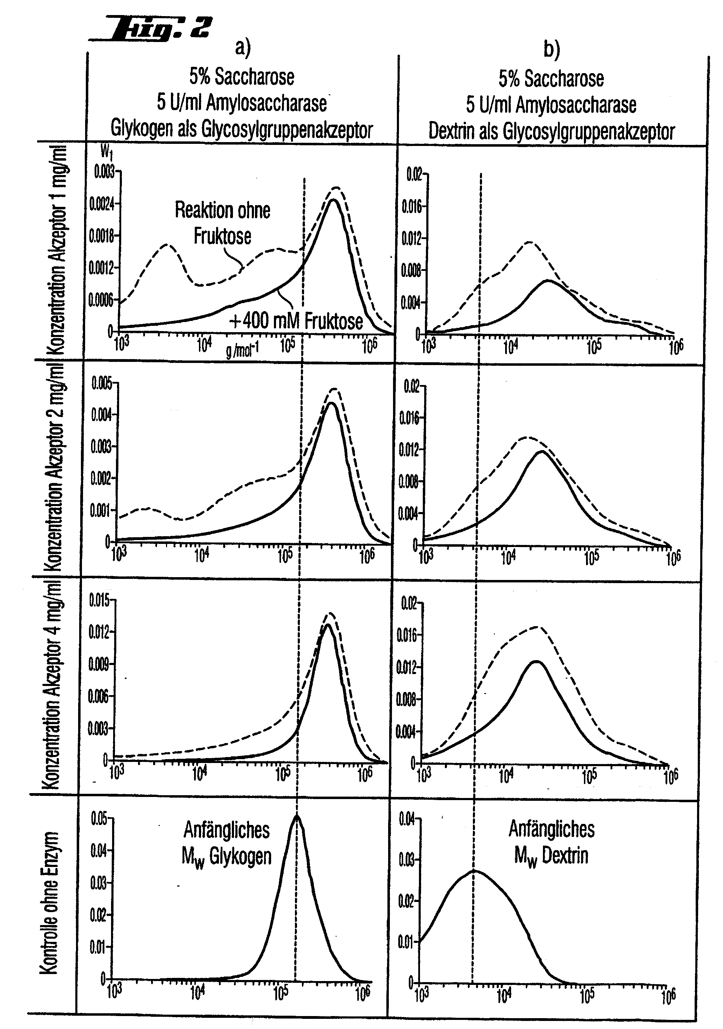

3.2 In einem weiteren Versuch wurde Amylosaccharase (5 U/ml) in 10 ml 0,1 M Natriumacetat-Puffer, pH 6,5, 0,02% Natriumazid, 5% Saccharose (w/v), bei 37°C mit Glykogen in verschiedenen Konzentrationen und in Anwesenheit von Fruktose (Anfangskonzentration 400mM) odr ohne Fruktose bis zum vollständigen Umsatz der Saccharose (mindestens 48 hr) inkubiert. Eine parallele Kontrollprobe ohne Enzym wurde unter ansonsten identischen Bedingungen behandelt. Die erhaltenen Polysaccharidprodukte wurden wie unter 3.1 beschrieben abzentrifugiert, gewaschen, gefriergetrocknet und mittels GPC analysiert. Die Ergebnisse sind graphisch in Fig. 2a dargestellt, wobei $W_i$ die oben genannte Bedeutung hat. Die senkrechte gepunktete Linie zeigt das Anfangsmolekuargewicht des als Glucosylgruppenakzeptor eingesetzten Glykogens.

Die Ergebnisse zeigen, daß die Polydispersität der erhaltenen Polysaccharide bei gleichbleibender Saccharosekonzentration und steigenden Glykogenkonzentrationen sinkt. In Anwesenheit von Fruktose wird eine weitere Abnahme der Polydispersität beobachtet.

3.3 Der unter 3.2 beschriebene Versuch wurde unter identischen Bedingungen wiederholt, jedoch wurde anstelle von Glykogen Dextrin (Sigma Nr. D-4894, Typ IV aus Kartoffeln, $M_W$ 6.650) eingesetzt. Nach Inkubation bei 37°C bis zum vollständigen Umsatz der Saccharose (mindestens 48 hr) in An- und Abwesenheit von Fruktose wurden die erhaltenen Polysaccharide wie oben beschrieben abzentrifugiert, gewaschen und gefriergetrocknet. Eine parallele Kontrollprobe ohne Enzym wurde unter ansonsten identischen Bedingungen behandelt und wie oben beschrieben aufgearbeitet und analysiert. Die Ergebnisse sind graphisch in Fig. 2 dargestellt, wobei $W_i$ die oben genannte Bedeutung hat. Die senkrechte gepunktete Linie zeigt das Anfangsmolekuargewicht des als Glucosylgruppenakzeptor eingesetzten Dextrins.

Die Ergebnisse zeigen, daß die Polydispersität der erhaltenen Polysaccharide bei gleichbleibender Saccharosekonzentration und steigenden Dextrinkonzentrationen sinkt. In Anwesenheit von Fruktose wird eine weitere Abnahme der Polydispersität beobachtet.

**Patentansprüche**

1. Verfahren zur Herstellung von $\alpha$-1,4-Glucanketten enthaltenden Polysacchariden, **dadurch gekennzeichnet, dass** ein Glucosylgruppenakzeptor durch Umsetzung mit Saccharose in Gegenwart einer Amylosaccharase und unter Zusatz von Fructose einer Kettenverlängerungsreaktion unterworfen wird.

2. Verfahren nach Anspruch 1, **dadurch gekennzeichnet, dass** die Menge an Glucosylgruppenakzeptor im Reaktionsgemisch so gewählt ist, dass das Molverhältnis von zur Kettenverlängerung zur Verfügung stehenden Enden des Glucosylgruppenakzeptors zu Saccharose wenigstens 1 :1000 und/oder das Gewichtsverhältnis von Glucosylgruppenakzeptor zu Saccharose wenigstens 1 : 50 beträgt.

3. Verfahren nach Anspruch 1 oder 2, **dadurch gekennzeichnet, dass** das Molverhältnis von zur Kettenverlängerung zur Verfügung stehenden Enden des Glucosylgruppenakzeptors zu Saccharose wenigstens 5 :1000.

4. Verfahren nach einem der vorstehenden Ansprüche, **dadurch gekennzeichnet, dass** die Fructose dem Reaktionsgemisch in einer Konzentration von wenigstens 10 mM zugesetzt wird.

5. Verfahren nach einem der vorstehenden Ansprüche, **dadurch gekennzeichnet, dass** die Fructose dem Reaktionsgemisch in einer Konzentration von wenigstens 50 mM und vorzugsweise von 100 bis 800 mM zugesetzt wird.

6. Verfahren nach einem der vorstehenden Ansprüche, **dadurch gekennzeichnet, dass** als Amylosaccharase eine Amylosaccharase aus Bakterien der Gattung *Neisseria* eingesetzt wird.

7. Verfahren nach Anspruch 6, **dadurch gekennzeichnet, dass** als Amylosaccharase eine Amylosaccharase aus Bakterien der Spezies *Neisseria polysaccharea* eingesetzt wird.

8. Verfahren nach einem der Ansprüche 1 bis 7, **dadurch gekennzeichnet, dass** als Glucosylgruppenakzeptor Dextrine, Amylose, Amylopektin oder Glykogen eingesetzt werden.

9. Anwendung des Verfahrens nach einem der Ansprüche 1 bis 8 zur Steuerung des Molekulargewichts bei der Herstellung von Polysacchariden.

10. Verwendung von $\alpha$-1,4-Glucanketten enthaltenden Polysacchariden erhältlich durch ein Verfahren nach einem der Ansprüche 1 bis 8 als Tablettenfüllstoff.

## Claims

1. Procedure for the manufacture of polysaccharides containing α-1,4-glucan chains, **characterised in that** a glucosyl group acceptor is subjected to a chain-lengthening procedure by reaction with saccharose in the presence of an amylosaccharase and with the addition of fructose.

2. Procedure in accordance with claim 1, **characterised in that** the amount of glucosyl group acceptor in the reaction mixture is such that the mol ratio of the ends of the glucosyl group acceptor available for chain-lengthening to saccharose is at least 1 : 1,000 and / or the weight ratio of glucosyl group acceptor to saccharose amounts to at least 1 : 50.

3. Procedure in accordance with claims 1 or 2, **characterised in that** the mol ratio of the ends of the glucosyl group acceptor available for chain-lengthening to saccharose is at least 5 : 1,000.

4. Procedure m accordance with one of the preceding claims, **characterised in that** the fructose is added to the reaction mixture in a concentration of at least 10 mM.

5. Procedure in accordance with one of the preceding claims, **characterised in that** the fructose is added to the reaction mixture in a concentration of at least 50 mM and, preferably, of between 100 and 800 mM.

6. Procedure in accordance with one of the preceding claims, **characterised in that** the amylosaccharase employed is an amylosaccharase consisting of bacteria of the species *Neisseria*.

7. Procedure in accordance with claim 6, **characterised in that** the amylosaccharase employed is an amylosaccharase consisting of bacteria of the species *Neisseria polysaccharea*.

8. Procedure in accordance with one of the claims 1 to 7, **characterised in that** dextrin, amylose, amylopectin or glycogen is employed as the glucosyl group acceptor.

9. Use of the procedure in accordance with one of the claims 1 to 8 to control the molecular weight during the manufacture of polysaccharides.

10. Use as a tablet filler of polysaccharides containing α-1,4-glucan chains obtained by means of a procedure in accordance with one of the claims 1 to 8.

## Revendications

1. Procédé pour la préparation de polysaccharides contenant des chaînes de α-1,4-glucane, **caractérisé en ce qu'**un accepteur de groupes glucosyles est soumis par transformation avec du saccharose en présence d'une amylosaccharase et par ajout de fructose à une réaction de prolongement de chaînes.

2. Procédé selon la revendication 1, **caractérisé en ce que** la quantité en accepteur de groupes glucosyles dans le mélange réactionnel est choisie de telle façon que le rapport molaire des extrémités de l'accepteur de groupes glucosyles libres pour le prolongement des chaînes par rapport au saccharose s'élève au moins à 1:1000 et/ou que le rapport en poids de l'accepteur de groupes glucosyles par rapport au saccharose s'élève au moins à 1:50.

3. Procédé selon la revendication 1 ou 2, **caractérisé en ce que** le rapport molaire des extrémités de l'accepteur de groupes glucosyles libres pour le prolongement des chaînes au saccharose s'élève au moins à 5:1000.

4. Procédé selon l'une des revendications précédentes, **caractérisé en ce que** le fructose est ajouté au mélange réactionnel dans une concentration d'au moins 10 mM.

5. Procédé selon l'une des revendications précédentes, **caractérisé en ce que** le fructose est ajouté au mélange réactionnel dans une concentration d'au moins 50 mM et de préférence de 100 à 800 mM.

6. Procédé selon l'une des revendications précédentes, **caractérisé en ce que** l'on utilise en tant qu'amylosaccharase une amylosaccharase de bactéries du genre *Neisseria*.

**7.** Procédé selon la revendication 6, **caractérisé en ce que** l'on utilise en tant qu'amylosaccharase une amylosaccharase de bactéries de l'espèce *Neisseria polysaccharea*.

**8.** Procédé selon l'une des revendications 1 à 7, **caractérisé en ce que** l'on utilise en tant qu'accepteur de groupes glucosyles la dextrine, l'amylose, l'amylopectine ou le glycogène.

**9.** Utilisation du procédé selon l'une des revendications 1 à 8 pour le contrôle du poids moléculaire par la préparation de polysaccharides.

**10.** Utilisation de polysaccharides contenant des chaînes de $\alpha$-1,4-glucane selon l'une des revendications 1 à 8 en tant que charge pour comprimés.

*Fig. 1*

EP 1 141 370 B1

Fig. 2